Europäisches Patentamt

European Patent Office

Office européen des brevets

(11) Publication number: 0 303 980
A2

# EUROPEAN PATENT APPLICATION

(21) Application number: 88113121.3

(22) Date of filing: 12.08.88

(51) Int. Cl.⁴: G01N 33/531 , G01N 33/543

Claims for the following Contracting State: ES.

The title of the invention has been amended (Guidelines for Examination in the EPO, A-III, 7.3).

(30) Priority: 20.08.87 JP 205069/87

(43) Date of publication of application:
22.02.89 Bulletin 89/08

(84) Designated Contracting States:
AT BE CH DE ES FR GB GR IT LI LU NL SE

(71) Applicant: HOECHST JAPAN LIMITED
10-16, 8-chome, Akasaka, Minato-ku
Tokyo(JP)

(72) Inventor: Jitsukawa, Tomofumi
Mezon-Yamataka C-201 787-1,
Ohaza-Minamiohtsuka
Kawagoe-shi Saitama-ken(JP)
Inventor: Nakajima, Satoko
Tanakaren Bldg.302 2-4-24, Minamidai
Kawagoe-shi Saitama-ken(JP)
Inventor: Watanabe, Hiroshi
1486-464, Ohaza-Ishizaka Hatoyama-machi
Hiki-gun Saitama-ken(JP)

(74) Representative: Klein, Otto, Dr. et al
Hoechst AG Zentrale Patentabteilung
Postfach 80 03 20
D-6230 Frankfurt am Main 80(DE)

(54) Carrier coated with antigen or antibody.

(57) A highly sensitive immunoassay agent can be obtained from a small amount of an antigen or antibody by fixing the antigen or antibody on a solid support in a solution in the presence of a protein different from said antigen or antibody. Suitable proteins are 1) serum proteins such as bovine serum albumin, human immunoglobulin and rabbit immunoglobulin; 2) sera themselves such as human serum and fetal calf serum and 3) other proteins such as human liver ferritin and human recombinant gamma interferon. Bovine serum albumin is especially preferable.

EP 0 303 980 A2

## Carrier of antigen or antibody

Background of the Invention

1. Field of the Invention

The present invention relates to a novel carrier for an antigen or antibody. The carrier of antigen or antibody according to the invention is advantageously used in immunoassay.

"Immunoassay" is a method for identifying or quantitatively determining an antigen or an antibody by the antigen-antibody reaction, which method has been widely employed in recent years in the clinical tests in hospitals and other institutions.

2. Description of Prior Art

The immunoassays now available include enzyme immunoassays, fluorescence immunoassays and the radioisotope-labeled antibody method (radioimmunoassay). In these methods, which are run with an enzyme-, fluorescence- and a radioisotope-label, respectively, a highly specific reaction between antigen and antibody is used in principle.

Although there are a variety of practical modes for the immunoassay, a plastic support such as polystyrene on which an antigen or an antibody is fixed by adsorption is most commonly used, for an example in the ELISA method (enzyme-linked immunosorbent assay).

In the ELISA method, an antigen (or antibody) is fixed on the inner surface of a vehicle such as a poly-styrene test tube and the like, and then reacted with an antibody (or antigen) contained in a sample. Subsequently, the antigen-antibody complex is reacted with a secondary antibody labeled with an enzyme such as a peroxidase that will form a color-developing reaction product, followed by measurement of the degree of the color development. Such an immunoassay is conducted, for example, by a direct method, an indirect method or a sandwich method but there are other test variants like competition assays etc. The detailed explanation may be found, for example, in the publications, "Yaku-ni-tatsu Meneki-jikkenho" (Applicable Methods for Immunological Experiments) (published by Kodansha, Tokyo, Japan) and "Meneki Seikagaku Kenkyuho" (Methods for Studying Immunological Biochemistry) (published by Tokyo Kagaku Dojin, Tokyo, Japan).

Heretofore, the inner surface of a test tube has been treated with a solution containing an antigen (or antibody) at a concentration of 5 μg/ml at lowest in order to fix the antigen (or antibody) on the inner surface of the test tube. This is because the result with an antigen (or antibody) concentration lower than 5 μg/ml cannot always be produced at satisfactory sensitivity. However, some antigens (or antibodies) are available only in a limited amount, and so the treatment at a lower concentration is desirable.

Brief Description of the Drawing

Fig. 1 is a graphic presentation of the results of Test Example 1 in which HRPO was supported on a polystyrene carrier.

The mark o in the figure indicates data for the support prepared in the absence of bovine serum albumin, and the mark ● indicates data for the support prepared in the presence of bovine serum albumin.

Fig. 2 is a graphic presentation of the results of Test Example 2 in which rIFN-γ was supported on a polystyrene carrier.

The mark o in the figure indicates data for the support prepared in the absence of bovine serum albumin, and the mark ● indicates data for the support prepared in the presence of bovine serum albumin.

Summary of the Invention

An object of the invention is to provide immunoassay agents with a high sensitivity formed with decreased amounts of reagents needed in the coating step by improving the method for binding reagents such as an antigen on a solid support.

The carrier of an antigen or antibody according to the invention is characterized by fixing an antigen or an antibody in solution on a solid support in the presence of a protein different from said antigen or antibody.

There is no limitation in the antigen of the invention and any proteins may be used which can be on a solid support and used as an antigen. Haptens comprising soluble liposaccharides may also be employed.

The "antibody" means any antibody against these antigens. Both polyclonal antibodies obtained from an animal serum immunized with an antigen and monoclonal antibodies produced by the hybrid cells between spleen cells of an immunized animal and myeloma cells can be used.

As solid support any of the synthetic-resin solid support heretofore used in immunoassay can be used. For example, solid supports made of a polystyrene, a surface-treated polystyrene or a polyvinyl compound are advantageously used. It is desirable that the solid support is formed as a test tube or wells if the number of the sample is large. It may be in the shape of a plate, a ball or the like.

In fixing the antigen (or antibody) on the solid support, the protein added to the antigen (or antibody) must be immunologically different from the antigen-antibody system to be used. "Immunologically different" means that the added protein does not compete or interact in a negative way with the antibody-antigen-complex formation. As such added protein there are mentioned 1) serum proteins such as bovine serum albumin, human immunoglobulin and rabbit immunoglobulin; 2) serums themselves such as human serum and fetal calf serum and 3) other proteins such as human liver ferritin and human recombinant gamma interferon. Bovine serum albumin is especially preferable.

In fixing an antigen (or antibody) on a solid support according to the invention, the antigen (or antibody) is dissolved to form a solution together with another protein, which solution is then contacted with a support. The concentration of the antigen (or antibody) employed is preferably in the range from about 20 μg/ml to 0.05 μg/ml, although it is variable depending upon the nature of the antigen (or antibody). The optimal concentration of the antigen (or antibody) to be fixed is variable depending upon the nature of the antigen (or antibody). The concentration of the protein coexisting with the antigen (or antibody) is also variable depending upon its nature and also upon the antigen (or antibody). It is preferably applied in the range from about 500 μg/ml to 0.1 μg/ml. In general, the total concentration of the antigen (or antibody) and the added protein is preferably about 5 μg/ml.

The solution of the antigen (or antibody) is preferably brought into contact with the solid support at room temperature for 30-120 min. or at 4°C overnight. Usually the contact will be sufficient at room temperature for 60 min. In order to block protein-binding sites remaining on the solid support on which the antigen (or antibody) has been supported, the support is treated with an appropriate protein by a conventional method. Whereas 1% bovine serum albumin is usually employed, 5% fetal calf serum is especially suitable. The supports thus obtained according to the invention can be utilized in the same way as the prior-art supports.

In the ELISA method, the support is contacted with a sample containing an antibody (or antigen) to be measured in an antigen-antibody reaction. The resulting antigen-antibody complex is subjected to a reaction with an enzyme-labeled antibody against the antibody (or antigen) to be measured, and subsequently to the measurement of color development by the enzyme.

Results of the invention will be described in the following Test Examples.

Test Example 1. Coating horse-radish peroxidase onto a solid support

A coating was made by the use of horse-radish peroxidase (called "HRPO" hereinbelow) on a polystyrene plastic well for ELISA manufactured by Sankoh Junyaku (Pure Chemical) Co., Ltd. (SJ-109-81) as follows:

(1) Coating in the absence of a different protein

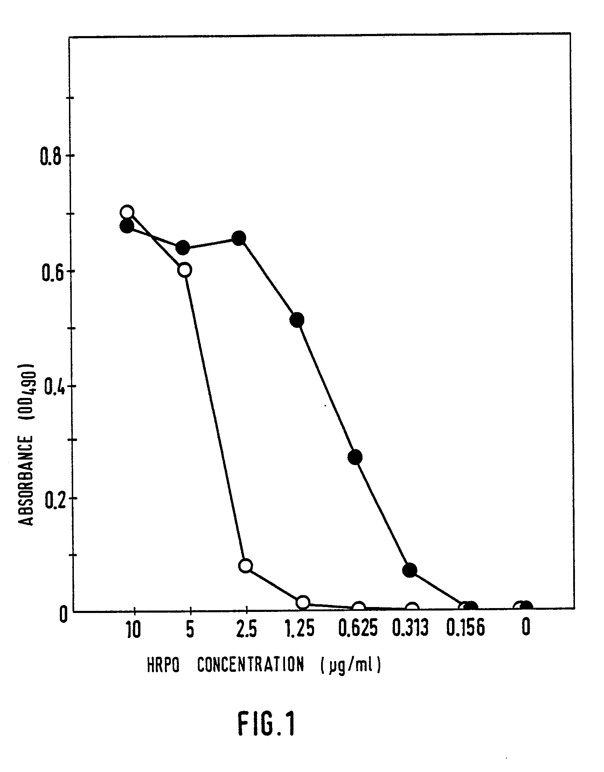

HRPO was diluted with phosphate-buffered saline (called "PBS" hereinbelow) in a double dilution method to prepare 10-, 5-, 2.5-, 1.25-, 0.625-. 0.313- and 0.156 μg/ml solutions. The solutions each in an amount of 60 μl were divided into wells and maintained at room temperature for 1 hour to effect coating. The coated wells were washed 5 times with PBS then a citrate buffer solution (pH 5.0, containing 0.006% $H_2O_2$) containing 1 mg/ml o-phenylenediamine was added for color development. After 20-40 min. 60 μl of 2M sulfuric acid was added to stop the color development reaction. Absorbance at 490 nm was measured with an ELISA autoreader (Dynatech MR580). The results are shown in Fig. 1.

(2) Coating in the presence of bovine serum albumin

HRPO was diluted with PBS in a double dilution method to prepare 20-, 10-, 5-, 2.5-, 1.25-, 0.625- and 0.313 μg/ml solutions. Each of the solutions was mixed in a 1:1 ratio in volume with PBS solution containing 5.6 μg/ml bovine serum albumin (called BSA hereinbelow) and the solutions each in an amount of 60 μl were divided into wells and maintained at room temperature for 1 hour to effect coating. After having washed 5 times with PBS, color development was effected in the same way as above and measured for absorbance at 490 nm. The results are shown in Fig. 1.

In the absence of BSA the HRPO coating was insufficient with HRPO at a concentration of 1.25 μg/ml or lower. In the presence of BSA, on the other hand, the coating was sufficient at a concentration of 0.313 μg/ml or higher after diluting with the BSA-containing PBS.

Test Example 2. Coating genetic recombinant interferon gamma onto a solid support

As an antigen was employed interferon gamma prepared by genetic engineering techniques (called "rIFN-γ" hereinbelow), which was coated on a polystyrene plastic well for ELISA manufactured by Sankoh Junyaku (Pure Chemical) Co., Ltd. (SJ-109-81) as follows:

(1) Coating in the absence of a different protein

rIFN-γ was diluted with PBS in a double dilution method to prepare 10-, 5-, 2.5-, 1.25-, 0.625-, 0.313- and 0.156 μg/ml solutions. The solutions each in an amount of 60 μl were pipetted into wells and maintained at room temperature for 1 hour to effect coating. The coated wells were washed 3 times with PBS. Into the wells there was pipetted 5% heat-inactivated fetal calf serum (called "FCS" hereinbelow) followed by standing at room temperature for 20 min. to block the remaining protein-binding sites. Washing was then made four times with PBS.

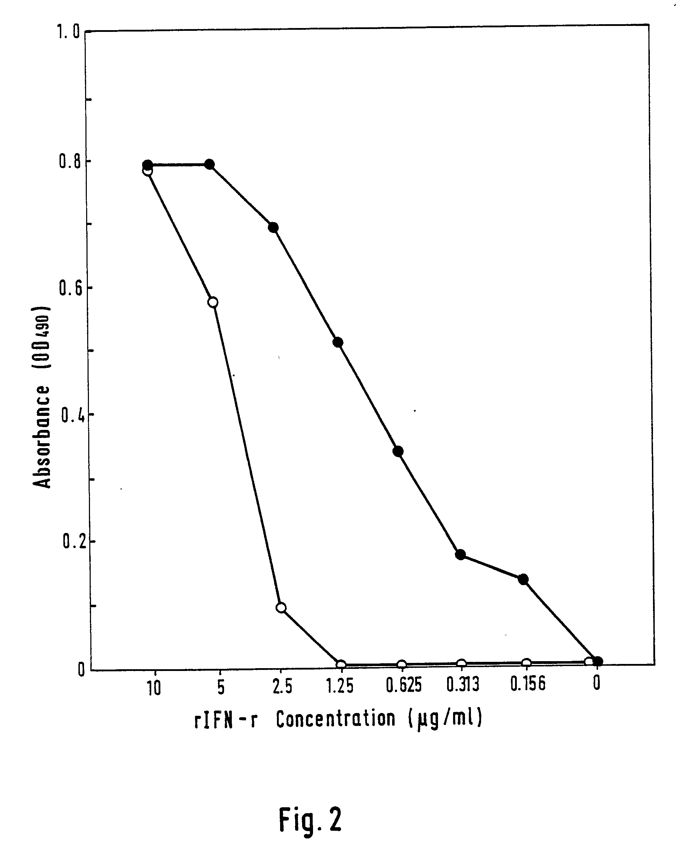

Into each well was filled 40 μl of 10 μg/ml solution of D1G2, a monoclonal antibody to IFN-Y (an antibody produced by hybridoma techniques which was obtained by immunizing a BALB/c mouse with natural IFN-γ and subjecting spleen cells of the mouse to cell fusion with myeloma cells in a conventional manner) followed by maintaining at room temperature for one hour and washing five times with PBS. Then, 40 μl of the 1:2000 dilution of a rabbit antibody against mouse immunoglobulins labeled with HRPO was added, followed by standing at room temperature for one hour. The wells were washed five times with PBS and then 60 μl of a citrate buffer solution (pH 5.0, containing 0.006% $H_2O_2$) containing 1 mg/ml o-phenylenediamine were added for color development. After 20-40 min. 60 μl of 2M sulfuric acid were added to stop the color development reaction. Absorbance at 490 nm was measured with an ELISA autoreader. The results are shown in Fig. 2.

(2) Coating in the presence of BSA

rIFN-γ was diluted with PBS in a double dilution method to prepare 20-, 10-, 5-, 2.5-, 1.25-, 0.625- and 0.313 μg/ml solutions. Each of the solutions was mixed in a 1:1 ratio with PBS containing 100 μg/ml BSA, and the mixed solution was pipetted into wells. Coating was effected by maintaining the solid support e.g. testplate at room temperature for 1 hour.

The reaction with the monoclonal antibody D1G2 was carried out in exactly the same way as above followed by color development and measurement of the absorbance made in the same way as above. The results are shown in Fig. 2.

In the absence of BSA, the coating was insufficient with rIFN-γ at a concentration of 1.25 μg/ml or lower. In the presence of BSA, the coating was sufficient at a concentration of 0.156 μg/ml or higher when diluted with the BSA-containing PBS

Example 1.

Polystyrene plastic wells were treated with a PBS containing 1 μg/ml of rIFN-γ and 30 μg/ml of human immunoglobulin G for one hour to support rIFN-γ on the inner surface of the wells. Antibody titer to rIFN-γ was measured with the resulting wells to find that the titer could be determined in approximately the same accuracy as with the plastic wells treated with 10 μg/ml of rIFN-γ.

Example 2.

Polystyrene plastic wells for ELISA were treated with a PBS containing 1.2 μg/ml of HRPO and 1 μg/ml of human immunoglobulin G for one hour to support HRPO on the inner surface of the wells. Measurement of the supported HRPO was made with the wells to find that it could be accomplished in approximately the same accuracy as with the plastic wells treated with 10 μg/ml of HRPO.

Example 3.

Polystyrene plastic wells for ELISA were treated with a PBS containing 3 μg/ml of human liver ferritin (called HLF hereinbelow) and 3 μg/ml of human immunoglobulin G for one hour to support HLF on the inner surface of the wells. Antibody titer to HLF was measured with the resulting wells to find that the titer could be determined in approximately the same accuracy as with the plastic wells treated with 20 μg/ml of HLF.

Example 4.

Polystyrene plastic wells for ELISA were treated with a PBS solution containing 2.5 μg/ml of HLF and 2 μg/ml of BSA for one hour. Antibody titer to HLF was measured with the resulting wells to find that the titer could be determined in approximately the same accuracy as with plastic wells treated with 10 μg/ml of HLF.

## Claims

1. A process for fixing an antigen or antibody and an immunologically different protein onto a solid carrier, characterized by contacting the carrier surface with a solution containing both the antigen or antibody and the immunologically different protein.

2. A process according to claim 1, characterized in that the immunologically different protein is a serum protein.

3. A process according to claim 2, characterized in that the serum protein is bovine serum albumin.

4. A coated carrier, obtained by a process according to one or more of the preceding claims.

5. The use of a carrier according to claim 4 in a diagnostic process.

Claims for the following contracting State: ES

1. A process for fixing an antigen or antibody and an immunologically different protein onto a solid carrier, characterized by contacting the carrier surface with a solution containing both the antigen or antibody and the immunologically different protein.

2. A process according to claim 1, characterized in that the immunologically different protein is a serum protein.

3. A process according to claim 2, characterized in that the serum protein is bovine serum albumin.

FIG.1

Fig. 2